# EUROPEAN PATENT APPLICATION

(11) **EP 4 834 956 A1**
(43) Date of publication of application: **04.11.2026**
(21) Application number: 25751932.2
(22) Date of filing: 31.01.2025
(51) Int. Cl.: C08F 20/38, A61K 6/15, A61K 6/891

(54) **THIOURETHANE (METH)ACRYLATE COMPOUND, MONOMER COMPOSITION, CURABLE COMPOSITION, AND DENTAL MATERIAL**

(30) Priority: 05.02.2024 JP 2024015856
(71) Applicant: Mitsui Chemicals, Inc., Tokyo 104-0028 (JP)
(72) Inventor: ABO, Eri, Tokyo 104-0028 (JP); KOHGO, Osamu, Tokyo 104-0028 (JP); KOSUGI, Yoko, Tokyo 104-0028 (JP)
(74) Representative: Hoffmann Eitle
(86) International application number: PCT/JP2025/003278
(87) International publication number: WO 2025/169863

(57) **Abstract**

A thiourethane (meth)acrylate compound includes two bonds each represented by the following Formula (X), two urethane bonds, and two (meth)acryloyl groups. In Formula (X), each of the two instances of * is a bonding position.

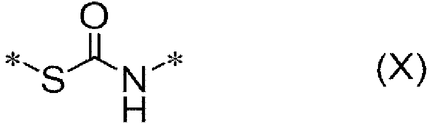

## Description

### Technical Field

The present disclosure relates to a thiourethane (meth)acrylate compound, a monomer composition, a curable composition, and a dental material.

### Background Art

A composition for a dental material such as a composite resin generally contains a composition for a dental material containing a monomer, a polymerization initiator, and the like.

There are cases in which a composition containing a polyfunctional methacrylate, which is a radical-polymerizable monomer, is used as a composition for a dental material, for example, in view of *in vivo* safety of the monomer to be contained, mechanical strength of a cured product, and abrasion resistance of a cured product.

As the composition containing a polyfunctional methacrylate, a composition containing UDMA (a urethane dimethacrylate with CAS number 72869-86-4), which is a polyfunctional methacrylate, is known (see, for example, Patent Document 1).

Patent Document 1: Japanese National-Phase Publication (JP-A) No. 2002-518419

### SUMMARY OF INVENTION

### Technical Problem

For example, in the field of dentistry, a cured product obtained by curing a composition containing a monomer may be required to have excellent bending strength.

However, the above-described UDMA, which has already been used in the field of dentistry, has room for improvement in terms of the bending strength of the cured product.

Therefore, a (meth)acrylate capable of imparting excellent bending strength to the cured product is desired.

A problem to be solved by one aspect of the present disclosure is to provide a thiourethane (meth)acrylate compound, a monomer composition, a curable composition, and a dental material that are capable of imparting excellent bending strength to a cured product.

### Solution to Problem

Specific means for solving the above-described problem are described below.
<1> A thiourethane (meth)acrylate compound, including:
   two bonds each represented by the following Formula (X);
   two urethane bonds; and
   two (meth)acryloyl groups: 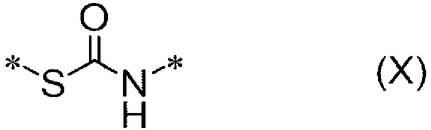 wherein, in Formula (X), each of two instances of * is a bonding position.
<2> The thiourethane (meth)acrylate compound according to <1>, which is a reaction product of a thiol compound (B) containing two mercapto groups, a (meth)acrylate compound (C) containing one hydroxy group and one (meth)acryloyloxy group, and an isocyanate compound (D) containing two isocyanate groups.
<3> The thiourethane (meth)acrylate compound according to <1> or <2>, which is a compound represented by the following Formula (A1): 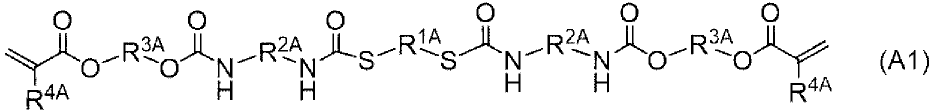 wherein, in Formula (A1):
   R^{1A} is a residue obtained by removing two mercapto groups from the thiol compound (B) containing two mercapto groups;
   R²⁴ is a residue obtained by removing two isocyanate groups from the isocyanate compound (D) containing two isocyanate groups;
   R^{3A} is a residue obtained by removing one hydroxy group and one (meth)acryloyloxy group from the (meth)acrylate compound (C) containing one hydroxy group and one (meth)acryloyloxy group;
   R^{4A} is a hydrogen atom or a methyl group;
   two instances of *R^{2A}* may be the same as or different from each other;
   two instances of *R^{3A}* may be the same as or different from each other; and
   two instances of *R^{4A}* may be the same as or different from each other.
<4> The thiourethane (meth)acrylate compound according to <2> or <3>, wherein the isocyanate compound (D) includes a compound represented by any one of the following Formulae (D1) to (D8): 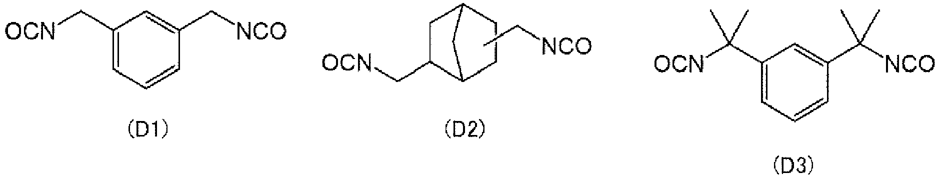 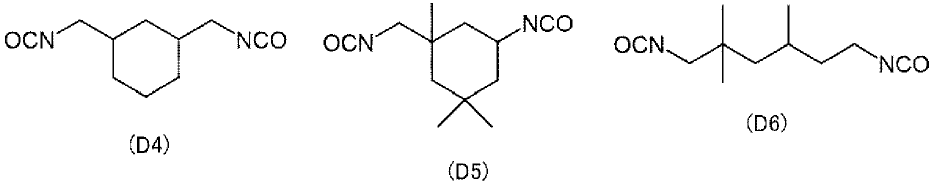 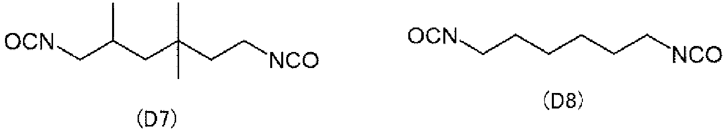
<5> The thiourethane (meth)acrylate compound according to any one of <2> to <4>, wherein the thiol compound (B) includes a compound represented by the following Formula (B1): 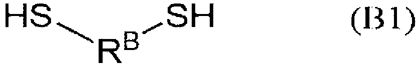 wherein, in Formula (B1), R^{B} is a divalent organic group having from 1 to 20 carbon atoms.
<6> The thiourethane (meth)acrylate compound according to any one of <2> to <5>, wherein the (meth)acrylate compound (C) includes a compound represented by the following Formula (C1): 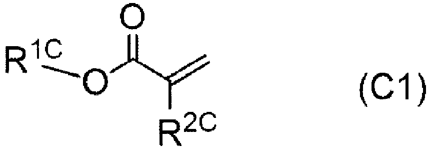 wherein, in Formula (C1):
   R^{1C} is an organic group having from 2 to 25 carbon atoms and containing one hydroxy group; and
   R^{2C} is a hydrogen atom or a methyl group.
<7> A monomer composition, including the thiourethane (meth)acrylate compound according to any one of <1> to <6>.
<8> The monomer composition according to <7>, wherein the monomer composition is for a dental material.
<9> A curable composition, including the thiourethane (meth)acrylate compound according to any one of <1> to <6>, and a polymerization initiator.
<10> A dental material, including the curable composition according to <9> or a cured product of the curable composition.

### Advantageous Effects of Invention

According to one aspect of the present disclosure, a thiourethane (meth)acrylate compound, a monomer composition, a curable composition, and a dental material that are capable of imparting excellent bending strength to a cured product are provided.

### BRIEF DESCRIPTION OF DRAWINGS

Fig. 1 is an infrared absorption spectrum of Monomer 1, which is a specific example of a thiourethane (meth)acrylate compound of the present disclosure.
Fig. 2 is an infrared absorption spectrum of Monomer 2, which is a specific example of the thiourethane (meth)acrylate compound of the present disclosure.
Fig. 3 is an infrared absorption spectrum of Monomer 3, which is a specific example of the thiourethane (meth)acrylate compound of the present disclosure.
Fig. 4 is an infrared absorption spectrum of Monomer 4, which is a specific example of the thiourethane (meth)acrylate compound of the present disclosure.
Fig. 5 is an infrared absorption spectrum of Monomer 5, which is a specific example of the thiourethane (meth)acrylate compound of the present disclosure.
Fig. 6 is an infrared absorption spectrum of Monomer 6, which is a specific example of the thiourethane (meth)acrylate compound of the present disclosure.
Fig. 7 is an infrared absorption spectrum of Monomer 7, which is a specific example of the thiourethane (meth)acrylate compound of the present disclosure.
Fig. 8 is an infrared absorption spectrum of Monomer 8, which is a specific example of the thiourethane (meth)acrylate compound of the present disclosure.
Fig. 9 is an infrared absorption spectrum of Monomer 9, which is a specific example of the thiourethane (meth)acrylate compound of the present disclosure.
Fig. 10 is an infrared absorption spectrum of Monomer 10, which is a specific example of the thiourethane (meth)acrylate compound of the present disclosure.
Fig. 11 is an infrared absorption spectrum of Monomer 11, which is a specific example of the thiourethane (meth)acrylate compound of the present disclosure.
Fig. 12 is an infrared absorption spectrum of Monomer 12, which is a specific example of the thiourethane (meth)acrylate compound of the present disclosure.
Fig. 13 is an infrared absorption spectrum of Monomer 13, which is a specific example of the thiourethane (meth)acrylate compound of the present disclosure.
Fig. 14 is an infrared absorption spectrum of Monomer 14, which is a specific example of the thiourethane (meth)acrylate compound of the present disclosure.
Fig. 15 is an infrared absorption spectrum of Monomer 15, which is a specific example of the thiourethane (meth)acrylate compound of the present disclosure.
Fig. 16 is an infrared absorption spectrum of Monomer 16, which is a specific example of the thiourethane (meth)acrylate compound of the present disclosure.
Fig. 17 is an infrared absorption spectrum of Monomer 17, which is a specific example of the thiourethane (meth)acrylate compound of the present disclosure.
Fig. 18 is an infrared absorption spectrum of Monomer 18, which is a specific example of the thiourethane (meth)acrylate compound of the present disclosure.
Fig. 19 is an infrared absorption spectrum of Monomer 19, which is a specific example of the thiourethane (meth)acrylate compound of the present disclosure.
Fig. 20 is an infrared absorption spectrum of Monomer 20, which is a specific example of the thiourethane (meth)acrylate compound of the present disclosure.
Fig. 21 is an infrared absorption spectrum of Monomer 21, which is a specific example of the thiourethane (meth)acrylate compound of the present disclosure.
Fig. 22 is an infrared absorption spectrum of Monomer 22, which is a specific example of the thiourethane (meth)acrylate compound of the present disclosure.

### DESCRIPTION OF EMBODIMENTS

In the present disclosure, a numerical range expressed using "to" indicates the range that includes the numerical values which are described before and after "to", as a minimum value and a maximum value, respectively.

In the present disclosure, the scope of the term "step" includes not only an independent step but also a step that cannot be clearly distinguished from other steps but that can achieve the intended purpose of the step of interest.

In the present disclosure, in a case in which plural substances corresponding to a particular component are present, the content of the component described in the present disclosure means the total content of the plural substances, unless otherwise specified.

In the present disclosure, the term "(meth)acryloyl group" means an acryloyl group and a methacryloyl group, and the term "(meth)acrylate" means acrylate and methacrylate.

### [Thiourethane (Meth)acrylate Compound (A)]

A thiourethane (meth)acrylate compound of the present disclosure (also referred to as the "thiourethane (meth)acrylate compound (A)" in the present disclosure) contains two bonds each represented by the following Formula (X) (i.e., thiourethane bonds), two urethane bonds, and two (meth)acryloyl groups:

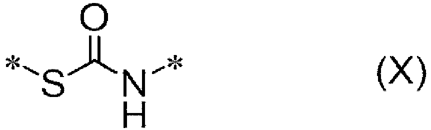

In Formula (X), each of the two instances of * is a bonding position.

Use of the thiourethane (meth)acrylate compound (A) (i.e., the thiourethane (meth)acrylate compound of the present disclosure) is capable of imparting excellent bending strength to a cured product.

The cured product as used herein means a cured product obtained by curing the thiourethane (meth)acrylate compound (A) alone or a composition containing the thiourethane (meth)acrylate compound (A).

That is, the cured product has an excellent bending strength (for example, breaking strength and elastic modulus in a bending test).

It is conceivable that this effect is produced by a combination of two bonds each represented by Formula (X) (i.e., thiourethane bonds), two urethane bonds, and two (meth)acryloyl groups contained in the thiourethane (meth)acrylate compound (A).

The molecular weight of the thiourethane (meth)acrylate compound (A) is not particularly limited as long as the compound contains two bonds represented by Formula (X), two urethane bonds, and two (meth)acryloyl groups.

The molecular weight of the thiourethane (meth)acrylate compound (A) is preferably 3000 or less, more preferably 2000 or less, still more preferably 1500 or less, and furthermore preferably 1300 or less.

The lower limit of the molecular weight of the thiourethane (meth)acrylate compound (A) is not particularly limited as long as the compound contains two bonds represented by Formula (X), two urethane bonds, and two (meth)acryloyl groups.

The thiourethane (meth)acrylate compound (A) is preferably a reaction product of a thiol compound (B), which contains two mercapto groups, a (meth)acrylate compound (C), which contains one hydroxy group and one (meth)acryloyloxy group, and an isocyanate compound (D), which contains two isocyanate groups.

In this case, a thiourethane bond is formed by a reaction between a mercapto group (also referred to as a thiol group) in the thiol compound (B) and an isocyanate group in the isocyanate compound (D), and a urethane bond is formed by a reaction between a hydroxy group in the (meth)acrylate compound (C) and an isocyanate group in the isocyanate compound (D).

In a case in which the thiourethane (meth)acrylate compound (A) is the reaction product, each of the thiol compound (B), the (meth)acrylate compound (C), and the isocyanate compound (D) may include only one kind of compound or two or more kinds of compound.

In Tables 1 and 2 in the after-mentioned Examples section, the thiol compound (B), the (meth)acrylate compound (C), and the isocyanate compound (D) are denoted as Thiol (B), (Meth)acrylate (C), and Isocyanate (D), respectively.

### <Compound Represented by Formula (A1)>

The thiourethane (meth)acrylate compound (A) (for example, a reaction product of the thiol compound (B), the (meth)acrylate compound (C), the isocyanate compound (D)) is preferably a compound represented by the following Formula (A1):

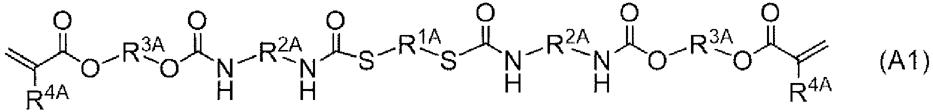

In Formula (A1):
R^{1A} is a residue obtained by removing two mercapto groups from the thiol compound (B) containing two mercapto groups;
R^{2A} is a residue obtained by removing two isocyanate groups from the isocyanate compound (D) containing two isocyanate groups;
R^{3A} is a residue obtained by removing one hydroxy group and one (meth)acryloyloxy group from the (meth)acrylate compound (C) containing one hydroxy group and one (meth)acryloyloxy group;
R^{4A} is a hydrogen atom or a methyl group;
two instances of *R^{2A}* may be the same as or different from each other;
two instances of *R^{3A}* may be the same as or different from each other; and
two instances of *R^{4A}* may be the same as or different from each other.

Hereinafter, a preferred aspect of raw materials (i.e., the thiol compound (B), the (meth)acrylate compound (C), and the isocyanate compound (D)) for preparing the thiourethane (meth)acrylate compound (A) (for example, the aforementioned reaction product and the aforementioned compound represented by Formula (A1)) will be described.

### <Thiol Compound (B)>

The thiol compound (B) is a compound containing two mercapto groups.

The molecular weight of the thiol compound (B) is preferably 1000 or less, more preferably 500 or less, and still more preferably 400 or less.

The thiol compound (B) preferably includes a compound represented by the following Formula (B1):

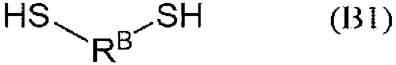

In Formula (B1), R^{B} is a divalent organic group having from 1 to 20 carbon atoms.

The divalent organic group represented by R^{B} may contain a hydrocarbon group.

The divalent organic group represented by R^{B} may further contain, for example, an ester bond, an ether bond, or a sulfide bond.

The proportion of the compound represented by Formula (B1) in the thiol compound (B) is preferably from 50 mass% to 100 mass%, more preferably from 60 mass% to 100 mass%, still more preferably from 80 mass% to 100 mass%, and furthermore preferably from 90 mass% to 100 mass%.

The compound represented by Formula (B1) preferably includes a thiol compound represented by any one of the following Formulae (B-1) to (B-5):

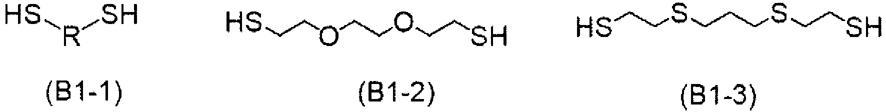

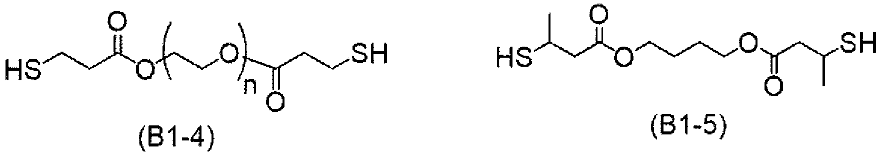

In Formula (B1-1), R is a divalent hydrocarbon group having from 1 to 20 carbon atoms.

The divalent hydrocarbon group represented by R is preferably an alkylene group having from 1 to 20 carbon atoms (preferably from 1 to 10 carbon atoms), an arylene group having from 1 to 20 carbon atoms (preferably from 1 to 10 carbon atoms) (for example, a phenylene group), an alkylene arylene group having from 1 to 20 carbon atoms (preferably from 1 to 10 carbon atoms) (for example, a methylene phenylene group), or an alkylene arylene alkylene group having from 1 to 20 carbon atoms (preferably from 1 to 10 carbon atoms) (for example, a methylene phenylene methylene group).

In Formula (B1-4), *n* is an integer from 1 to 10.

The total proportion of thiol compounds represented by Formulae (B-1) to (B-5) in the thiol compound (B) is preferably from 50 mass% to 100 mass%, more preferably from 60 mass% to 100 mass%, still more preferably from 80 mass% to 100 mass%, and furthermore preferably from 90 mass% to 100 mass%.

### <(Meth)acrylate Compound (C)>

The (meth)acrylate compound (C) is a compound containing one hydroxy group and one (meth)acryloyloxy group.

The molecular weight of the (meth)acrylate compound (C) is preferably 1000 or less, more preferably 500 or less, and still more preferably 400 or less.

The (meth)acrylate compound (C) preferably includes a compound represented by the following Formula (C1):

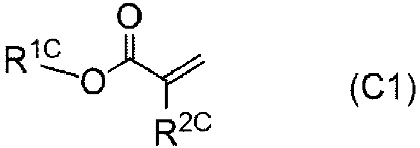

In Formula (C1), R^{1C} is an organic group having from 2 to 25 carbon atoms and containing one hydroxy group; and R^{2C} is a hydrogen atom or a methyl group.

The divalent organic group represented by R^{1C} may contain a hydroxy group and a hydrocarbon group.

The divalent organic group represented by R^{B} may further contain, for example, an ester bond, an ether bond, or a sulfide bond.

The proportion of the compound represented by Formula (C1) in the (meth)acrylate compound (C) is preferably from 50 mass% to 100 mass%, more preferably from 60 mass% to 100 mass%, still more preferably from 80 mass% to 100 mass%, and furthermore preferably from 90 mass% to 100 mass%.

Examples of the (meth)acrylate compound (C) include 2-hydroxyethyl (meth)acrylate, 2-hydroxypropyl (meth)acrylate, 2-hydroxybutyl (meth)acrylate, 4-hydroxybutyl (meth)acrylate, 2-hydroxy-3-phenoxypropyl (meth)acrylate, and 1,4-cyclohexanedimethanol mono(meth)acrylate.

The (meth)acrylate compound (C) preferably includes at least one selected from the group consisting of 2-hydroxyethyl acrylate (HEA), 2-hydroxyethyl methacrylate (HEMA), 2-hydroxypropyl acrylate (HPA), 2-hydroxypropyl methacrylate (HPMA), 2-hydroxybutyl acrylate (2HBA), 2-hydroxybutyl methacrylate (2HBMA), 4-hydroxybutyl acrylate (4HBA), 4-hydroxybutyl methacrylate (4HBMA), 2-hydroxy-3-phenoxypropyl acrylate, 2-hydroxy-3-phenoxypropyl methacrylate, 1,4-cyclohexanedimethanol monoacrylate, and 1,4-cyclohexanedimethanol monomethacrylate.

### <Isocyanate Compound (D)>

The isocyanate compound (D) is a compound containing two isocyanate groups.

The molecular weight of the isocyanate compound (D) is preferably 1000 or less, more preferably 500 or less, and still more preferably 400 or less.

The isocyanate compound (D) preferably includes a compound represented by any one of the following Formulae (D1) to (D8).

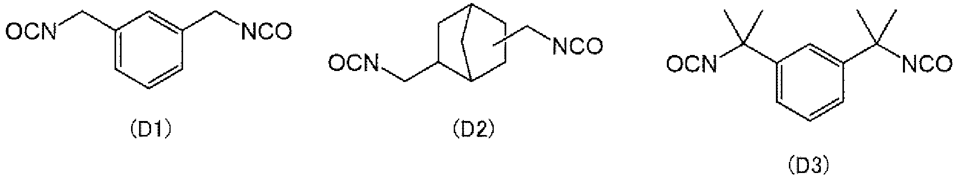

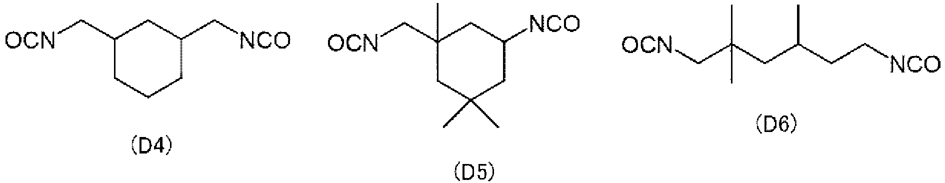

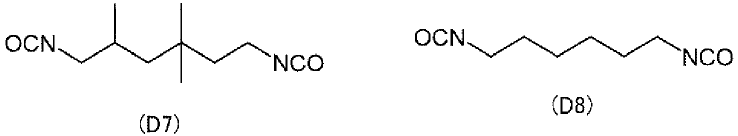

The total proportion of compounds represented by Formulae (D1) to (D8) in the isocyanate compound (D) is preferably from 50 mass% to 100 mass%, more preferably from 60 mass% to 100 mass%, still more preferably from 80 mass% to 100 mass%, and furthermore preferably from 90 mass% to 100 mass%.

### <Example of Method of Preparing Thiourethane (Meth)acrylate Compound (Preparation Method X)>

Hereinafter, an example of a method of preparing the thiourethane (meth)acrylate compound (A) (hereinafter also referred to as "preparation method X") will be described.

The preparation method X includes a reaction step of reacting the thiol compound (B), the (meth)acrylate compound (C), and the isocyanate compound (D) to prepare the thiourethane (meth)acrylate compound (A).

The preparation method X may include other steps if necessary.

According to the preparation method X, the thiourethane (meth)acrylate compound (A) (for example, the compound represented by Formula (A1)) is prepared as a reaction product of the thiol compound (B), the (meth)acrylate compound (C), and the isocyanate compound (D).

In the preparation method X, the molar ratio of mercapto groups included in the total charge amount of the thiol compound (B) to isocyanate groups included in the total charge amount of the isocyanate compound (D) (hereinafter also referred to as "input molar ratio [SH/NCO]" or simply "SH/NCO") is preferably from 0.01 to 0.50, more preferably from 0.02 to 0.40, and preferably from 0.03 to 0.30.

In the preparation method X, the molar ratio of hydroxy groups included in the total charge amount of the (meth)acrylate compound (C) to isocyanate groups included in the total charge amount of the isocyanate compound (D) (hereinafter also referred to as the "input molar ratio [OH/NCO]" or simply "OH/NCO"), i.e., the molar ratio of OH in the (meth)acrylate compound (C) to NCO, is set in consideration of the molar ratio of SH in the thiol compound (B) such that residual unreacted NCO of the isocyanate compound (D) is minimized as much as possible in the final post-synthesis thiourethane (meth)acrylate compound (A). For example, the input molar ratio [OH/NCO] is preferably from 0.50 to 1.50, more preferably from 0.60 to 0.99, and still more preferably from 0.70 to 0.97.

In the preparation method X, the molar ratio of the sum of mercapto groups included in the total charge amount of the thiol compound (B) and hydroxy groups included in the total charge amount of the (meth)acrylate compound (C) to isocyanate groups included in the total charge amount of the isocyanate compound (D) (hereinafter also referred to as the "input molar ratio [(SH + OH)/NCO]" or simply "(SH + OH)/NCO") is preferably from 1.00 to 2.00, more preferably from 1.00 to 1.50, and still more preferably from 1.00 to 1.30.

The reaction in the preparation method X may be performed solvent-free, or performed in a solvent.

As the solvent, a known solvent can be used as long as it is a solvent inert to the reaction.

Examples of the solvent include hydrocarbon solvents such as n-hexane, benzene, toluene, and xylene, ketone solvents such as acetone, methyl ethyl ketone, and methyl isobutyl ketone, ester solvents such as ethyl acetate and butyl acetate, ether solvents such as diethyl ether, tetrahydrofuran, and dioxane, halogen-containing solvents such as dichloromethane, chloroform, carbon tetrachloride, 1,2-dichloroethane, and perchlene, and polar solvents such as N,N-dimethylformamide, N,N-dimethylacetamide, N,N-dimethylimidazolidinone, dimethyl sulfoxide, and sulfolane.

These solvents may be used singly, or in combination of two or more thereof.

### (Catalyst)

A catalyst may be used in the foregoing reaction in the preparation method X.

Examples of the catalyst include:
organic tin compounds such as dibutyltin dilaurate, dibutyltin dioctate, and tin octanoate;
organometallic compounds other than tin such as copper naphthenate, cobalt naphthenate, zinc naphthenate, zirconium acetylacetonate, iron acetylacetonate, and germanium acetylacetonate; and
amine compounds such as triethylamine, 1,4-diazabicyclo[2.2.2]octane, 2,6,7-trimethyl-1-diazabicyclo[2.2.2]octane, 1,8-diazabicyclo[5.4.0]undecene, N,N-dimethylcyclohexylamine, pyridine, N-methylmorpholine, N,N,N',N'-tetramethylethylenediamine, N,N,N',N'-tetramethyl-1,3-butanediamine, N,N,N',N'-pentamethyldiethylenetriamine, N,N,N',N'-tetra(3-dimethylaminopropyl)-methanediamine, N,N'-dimethylpiperazine, and 1,2-dimethylimidazole and salts thereof; and
trialkylphosphine compounds such as tri-n-butylphosphine, tri-n-hexylphosphine, tricyclohexylphosphine, and tri-n-octylphosphine.

Among them, dibutyltin dilaurate and tin octanoate are preferable.

The amount of the catalyst to be used may be from 0.001 mass% to 1.0 mass%, or may be from 0.01 mass% to 0.5 mass%, with respect to the total of the thiol compound (B), the (meth)acrylate compound (C), and the isocyanate compound (D).

The reaction temperature is not particularly limited, and is, for example, from 20°C to 120°C, preferably from 30°C to 100°C, and still more preferably from 50°C to 100°C.

Since the reaction time depends on the conditions such as the reaction temperature, the reaction time is not particularly limited, and is, for example, from 5 minutes to 50 hours.

Examples of a method of confirming the end point of the reaction include analysis by HPLC (high performance liquid chromatography).

A polymerization inhibitor may be used in the reaction in the preparation method X.

Examples of the polymerization inhibitor include dibutylhydroxytoluene (BHT), hydroquinone (HQ), hydroquinone monomethyl ether (MEHQ), and phenothiazine (PTZ).

The used amount of the polymerization inhibitor may be from 0.001 mass% to 0.5 mass%, may be from 0.002 mass% to 0.3 mass%, or may be from 0.005 mass% to 0.3 mass%, with respect to the total of the thiol compound (B), the (meth)acrylate compound (C), and the isocyanate compound (D).

### [Monomer Composition]

A monomer composition of the present disclosure contains at least one thiourethane (meth)acrylate compound (A) (i.e., the thiourethane (meth)acrylate compound of the present disclosure).

Since the monomer composition of the present disclosure contains at least one thiourethane (meth)acrylate compound (A), the monomer composition exerts the same effect as the effect exerted by the thiourethane (meth)acrylate compound (A) (i.e., the effect in terms of imparting excellent bending strength (for example, breaking strength and elastic modulus in a bending test) to a cured product).

The content of the thiourethane (meth)acrylate compound (A) with respect to the total amount of the monomer composition of the present disclosure may be 10 mass% or more, 30 mass% or more, 50 mass% or more, 60 mass% or more, 80 mass% or more, or 90 mass% or more.

The upper limit of the content of the thiourethane (meth)acrylate compound (A) with respect to the total amount of the monomer composition of the present disclosure is, for example, 99 mass%, 95 mass%, or 90 mass%.

For example, the monomer composition of the present disclosure may be a composition composed of the thiourethane (meth)acrylate compound (A) in a content of 90 mass% or more and impurities.

The monomer composition of the present disclosure may contain a (meth)acrylate compound (hereinafter also referred to as "(meth)acrylate compound (E)") other than the thiourethane (meth)acrylate compound (A).

Examples of the (meth)acrylate compound (E) include neopentyl di(meth)acrylate, 1,4-butanediol di(meth)acrylate, 1,6-hexanediol di(meth)acrylate, 1,8-octanediol di(meth)acrylate, 1,9-nonanediol di(meth)acrylate, 1,10-decanediol di(meth)acrylate, tricyclodecanedimethanol di(meth)acrylate, ethylene glycol di(meth)acrylate, diethylene glycol di(meth)acrylate, triethylene glycol di(meth)acrylate, tetraethylene glycol di(meth)acrylate, polyethylene glycol di(meth)acrylate, tripropylene glycol di(meth)acrylate, tetrapropylene glycol di(meth)acrylate, polypropylene glycol di(meth)acrylate, 2,2-bis[4-(3-(meth)acryloyloxy-2-hydroxypropoxy)phenyl]propane, ethylene oxide-modified bisphenol A di(meth)acrylate, propylene oxide-modified bisphenol A di(meth)acrylate, and 2,2,4-trimethylhexamethylene bis(2-carbamoyloxyethyl) dimethacrylate (urethane dimethacrylate: UDMA). The (meth)acrylate compound (E) may be used singly, or in combination of two or more thereof.

For example, in order to adjust the viscosity of the monomer composition to a low viscosity, a viscosity adjusting monomer such as triethylene glycol dimethacrylate may be used.

In a case in which the viscosity adjusting monomer and the thiourethane (meth)acrylate (A) are used together, the viscosity adjusting monomer and the thiourethane (meth)acrylate (A) may be used in a mass ratio of from 1:0.8 to 1:1.2 or in a mass ratio of from 1:0.8 to 1:4.0 (preferably a mass ratio of from 1:1.1 to 1:4.0).

In a case in which the monomer composition of the present disclosure contains the (meth)acrylate compound (E), the proportion of the (meth)acrylate compound (E) in the monomer composition is preferably from 10 mass% to 80 mass%, more preferably from 20 mass% to 70 mass%, and still more preferably from 20 mass% to 60 mass%.

In a case in which the monomer composition of the present disclosure contains the (meth)acrylate compound (E), the proportion of the thiourethane (meth)acrylate (A) in the monomer composition is preferably from 20 mass% to 90 mass%, more preferably from 30 mass% to 80 mass%, and still more preferably from 40 mass% to 80 mass%.

The use applications of the monomer composition of the present disclosure are not particularly limited.

The monomer composition of the present disclosure is preferably a monomer composition for a dental material.

Specific examples of the dental material will be described later.

### <Curable Composition>

A curable composition of the present disclosure contains the thiourethane (meth)acrylate compound (A) (i.e., the thiourethane (meth)acrylate compound of the present disclosure) and a polymerization initiator.

The curable composition of the present disclosure may contain other components (for example, a (meth)acrylate compound (E)).

Since the curable composition of the present disclosure contains at least one thiourethane (meth)acrylate compound (A), the curable composition exerts the same effect as the effect exerted by the thiourethane (meth)acrylate compound (A) (i.e., the effect in terms of imparting excellent bending strength (for example, breaking strength and elastic modulus in a bending test) to a cured product).

The use applications of the curable composition of the present disclosure are not particularly limited.

The curable composition of the present disclosure is preferably a curable composition for a dental material.

Specific examples of the dental material will be described later.

The proportion of the thiourethane (meth)acrylate compound (A) in the curable composition of the present disclosure is preferably from 5 mass% to 95 mass%, more preferably from 10 mass% to 90 mass%, still more preferably from 20 mass% to 80 mass%, and furthermore preferably from 30 mass% to 70 mass%.

The curable composition of the present disclosure contains at least one polymerization initiator.

As the polymerization initiator, for example, a general polymerization initiator used in the field of dentistry can be used.

The polymerization initiator in the case of performing polymerization at ordinary temperature is, for example, preferably a redox polymerization initiator which includes an oxidizing agent and a reducing agent in combination. In a case in which the redox polymerization initiator is used, the oxidizing agent and the reducing agent may be in separately packaged forms, and may be mixed immediately before use.

The oxidizing agent is not particularly restricted, and examples thereof include organic peroxides such as diacyl peroxides (such as benzoyl peroxide), peroxy esters (such as t-butyl peroxy benzoate), dialkyl peroxides (such as dicumyl peroxide), peroxyketals (such as 1,1-bis(t-butylperoxy)-3,3,5-trimethylcyclohexane), ketone peroxides (such as methyl ethyl ketone peroxide), and hydroperoxides (t-butyl hydroperoxide).

The reducing agent is not particularly limited, and a tertiary amine (such as N,N-dimethylaniline) is usually used.

Examples of redox polymerization initiators that can be used include, in addition to these organic peroxide/amine-based redox polymerization initiators, redox polymerization initiators such as a cumene hydroperoxide/thiourea-based redox polymerization initiator, an ascorbic acid/Cu²⁺ salt-based redox polymerization initiator, and an organic peroxide/amine/sulfinic acid (or a salt thereof)-based redox polymerization initiator. Tributylborane or organic sulfinic acid, for example, is also suitable for use as a polymerization initiator.

In a case in which thermal polymerization by heating is performed, a polymerization initiator such as a peroxide or an azo-containing compound is preferable.

The peroxide is not particularly limited, and examples thereof include benzoyl peroxide, t-butyl hydroperoxide, and cumene hydroperoxide. The azo-containing compound is not particularly limited, and examples thereof include azobisisobutyronitrile.

In a case in which photopolymerization by visible light irradiation is performed, a redox initiator of, for example, α-diketone/tertiary amine, α-diketone/aldehyde, or α-diketone/mercaptan is preferable.

The photopolymerization initiator is not particularly limited, and examples thereof include an α-diketone/reducing agent, a ketal/reducing agent, and a thioxanthone/reducing agent.

Examples of the α-diketone include camphorquinone.

Examples of the ketal include benzyl dimethyl ketal.

Examples of the thioxanthone include 2-chlorothioxanthone.

Examples of the reducing agent include a tertiary amine (such as Michler Ketone), aldehydes (such as citronellal); and a compound having a thiol group (such as 2-mercaptobenzoxazole). A system such as an α-diketone/organic peroxide/reducing agent, which includes an organic peroxide added to a redox initiator as described above, is also suitable for use.

In a case in which photopolymerization is performed by ultraviolet irradiation, a photopolymerization initiator such as benzoin alkyl ether or benzyl dimethyl ketal is preferable. Photopolymerization initiators such as (bis)acylphosphine oxides are also suitable for use.

Examples of the (bis)acylphosphine oxides include acylphosphine oxides (such as 2,4,6-trimethylbenzoyl diphenylphosphine oxide), and bisacylphosphine oxides (such as bis-(2,6-dichlorobenzoyl)phenylphosphine oxide).

A (bis)acylphosphine oxide photopolymerization initiator, such as those described above, may be used alone or used together with a reducing agent such as any of various amines, aldehydes, mercaptans, or sulfinates. The (bis)acylphosphine oxide photopolymerization initiator may be used together with the photopolymerization initiator for visible light.

A polymerization initiator may be used with reference to, for example, WO 2021/29406 A1, WO 2019/107323 A1, or WO 2020/040141 A1.

The proportion of the polymerization initiator in the curable composition of the present disclosure is preferably from 0.01 mass% to 20 mass% and more preferably from 0.1 mass% to 5 mass%.

The curable composition of the present disclosure may contain at least one filler.

As the filler, a general filler used in the field of dentistry can be used.

Fillers are generally roughly classified into organic fillers and inorganic fillers.

Examples of organic fillers include fine powders of polymethyl methacrylate, polyethyl methacrylate, a methyl methacrylate-ethyl methacrylate copolymer, crosslinked polymethyl methacrylate, crosslinked polyethyl methacrylate, an ethylene-vinyl acetate copolymer, and a styrene-butadiene copolymer.

Examples of inorganic fillers include fine powders of various types of glasses (mainly composed of silicon dioxide and, if necessary, containing an oxide of a heavy metal, boron, aluminum, or the like), various ceramics, diatomaceous earth, kaolin, clay minerals (such as montmorillonite), activated clay, synthetic zeolite, mica, calcium fluoride, ytterbium fluoride, calcium phosphate, barium sulfate, zirconium dioxide, titanium dioxide, and hydroxyapatite. Specific examples of such inorganic fillers include barium borosilicate glass, strontium boroaluminosilicate glass, lanthanum glass, fluoroaluminosilicate glass, and boroaluminosilicate glass.

A filler may be used with reference to, for example, WO 2021/29406 A1, WO 2019/107323 A1, or WO 2020/040141 A1.

The blending amount of filler is preferably from 10 parts by mass to 2000 parts by mass, more preferably from 50 parts by mass to 1000 parts by mass, and still more preferably from 100 parts by mass to 600 parts by mass, with respect to 100 parts by mass of all components, other than filler, contained in the curable composition.

The curable composition of the present disclosure may contain components other than the above components.

Examples of the other components include a polymerization inhibitor, a colorant (such as a pigment or a dye), a reinforcing material (such as a fiber), a bactericide, a disinfectant, a stabilizer, and a preservative.

The curable composition of the present disclosure can be cured under appropriate conditions in the polymerization manner for the polymerization initiator described above. For example, in the case of a curable composition of the present disclosure that contains a photopolymerization initiator operable under visible light irradiation, a desired cured product can be obtained by processing the curable composition into a predetermined shape and then irradiated the curable composition with visible light for a predetermined time using a known photoirradiation device. Conditions such as irradiation intensity may be appropriately changed according to curability of the curable composition. The cured product that has been cured by photoirradiation such as visible light irradiation may further be subjected to heat treatment under appropriate conditions so as to improve the mechanical properties of the cured product.

### [Dental Material]

A dental material of the present disclosure includes the curable composition of the present disclosure or a cured product of the curable composition of the present disclosure.

The dental material of the present disclosure in an aspect in which the dental material includes the curable composition of the present disclosure is, for example, a composite resin for filling a carious cavity. In this case, the purpose can be fulfilled by filling a prepared tooth cavity in the oral cavity with a composite resin for filling a carious cavity, and then photocuring the composite resin using a known photoirradiation device.

The dental material of the present disclosure in an aspect in which the dental material includes the curable composition of the present disclosure may be a composite resin for dental crown. In this case, a desired dental crown material can be obtained by processing the composite resin for dental crown into an appropriate shape, and then photocuring the composite resin using a known photoirradiation device, and further, performing heat treatment under predetermined conditions.

The curable composition of the present disclosure may be used in dental treatment.

A dental treatment method using the curable composition of the present disclosure (or a dental material containing the curable composition) may include a step of polymerizing the curable composition of the present disclosure in the oral cavity to obtain a cured product.

The curable composition of the present disclosure (or a dental material containing the curable composition) when used for obtaining a cured product by polymerization in the oral cavity is suitable as, for example, a dental adhesive resin cement or a composite resin for filling restoration.

A dental treatment method using the curable composition of the present disclosure (or a dental material containing the curable composition) may include a step of polymerizing the curable composition of the present disclosure outside the oral cavity so as to obtain a cured product, and a step of applying the cured product in the oral cavity.

The step of polymerizing the curable composition of the present disclosure outside the oral cavity so as to obtain a cured product may be a step of polymerizing the curable composition of the present disclosure in a casting mold to obtain a cured product.

The cured product obtained by polymerizing the curable composition of the present disclosure outside the oral cavity may be processed as necessary, and the processed cured product may be applied in the oral cavity.

The cured product obtained by polymerizing the curable composition of the present disclosure outside the oral cavity (or a dental material containing the cured product) is suitable as, for example, a resin block for CAD/CAM, temporary crown, or an artificial tooth.

The curable composition of the present disclosure and the dental material of the present disclosure can be favorably used as, for example, a dental restorative material, a denture base resin, a denture base lining material, an impression material, a dental luting material (such as resin cement or resin-added glass ionomer cement), a dental adhesive (such as an orthodontic adhesive or a cavity coating adhesive), a tooth fissure sealant, a resin block for CAD/CAM, temporary crown, or an artificial tooth material.

In a case in which dental restorative materials are classified based on their application ranges, dental restorative materials can be classified into composite resins for a dental crown, composite resins for filling a carious cavity, composite resins for abutment construction, composite resins for filling restoration, and the like.

Among these, the curable composition of the present disclosure and the dental material of the present disclosure are particularly suitable for dental restorative materials such as composite resins.

### EXAMPLES

Hereinafter, examples in the present disclosure will be described; however, the present disclosure is not limited by the following examples.

Hereinafter, the simple notation "monomer" means a (meth)acrylate compound (specifically, thiourethane (meth)acrylate compound or comparative (meth)acrylate compound) unless otherwise specified.

Abbreviations of compounds used in the examples are shown below.

### <Isocyanate Compound (D)>

XDI: m-xylylene diisocyanate (compound (D1) described above)
TMXDI: 1,3-tetramethylxylylene diisocyanate (compound (D3) described above)
NBDI: mixture of 2,5-bis(isocyanatomethyl)bicyclo[2.2.1]heptane and 2,6-bis(isocyanatomethyl)bicyclo[2.2.1]heptane (compound (D2) described above)
TMHDI: trimethylhexamethylene diisocyanate (mixture of 2,2,4-substitution product (compound (D6) described above) and 2,4,4-substitution product (compound (D7) described above))

### <Thiol Compound (B)>

T1: 3,6-dioxa-1,8-octanedithiol (compound (B1-2) described above)
T2: 1,4-butanedithiol (compound (B1-1) described above)
T3: 1,8-octanedithiol (compound (B1-1) described above)
T4: bis(3-mercaptopropionic acid)ethylene glycol (compound (B1-4) described above)
T5: 3,7-dithia-1,9-nonanedithiol (compound (B1-3) described above)

### <(Meth)Acrylate Compound (C)>

4HBA: 4-hydroxybutyl acrylate
2HBMA: 2-hydroxybutyl methacrylate
HPMA: hydroxypropyl methacrylate
HPA: hydroxypropyl acrylate
HEA: 2-hydroxyethyl acrylate
HEMA: 2-hydroxyethyl methacrylate

### <(Meth)Acrylate Compound>

TEGDMA: triethylene glycol dimethacrylate
UDMA: 2,2,4-trimethylhexamethylene bis(2-carbamoyloxyethyl) dimethacrylate

### <Urethanization Catalyst>

DiBTSn: dibutyltin dilaurate

### <Polymerization Initiator>

CQ: (±)-camphorquinone
BEDB: 2-butoxyethyl 4-(dimethylamino) benzoate
LUPEROX 531M80 (1,1-di(t-amylperoxy)cyclohexane; manufactured by ARKEMA Yoshitomi, Ltd.)

### <Polymerization Inhibitor>

BHT: 2,6-di-tert-butyl-p-cresol

### <Preparation of Test Piece for Bending Test by Photopolymerization>

TEGDMA (4 parts by mass) was added to each monomer (specifically, each monomer shown in the column with a heading "6 (parts by mass)" in Tables 3 and 4) (6 parts by mass) to be evaluated, and the mixture was stirred at 50°C until it became uniform, thereby obtaining a monomer composition.

CQ (0.03 parts by mass) and BEDB (0.03 parts by mass) were added to the obtained monomer composition, and the mixture was stirred at room temperature until it became uniform, thereby obtaining a curable composition for photopolymerization.

The obtained curable composition for photopolymerization was placed in a stainless steel mold of 2 mm × 2 mm × 25 mm, and each side thereof was irradiated with light for 3 minutes, i.e., for 6 minutes as a total of both sides, using a visible light irradiation device (α LIGHT V, manufactured by J. MORITA CORP.). Thus, the monomers in the curable composition were photopolymerized, so that the curable composition was photocured, and a cured product was obtained.

The obtained cured product was immersed in distilled water in a sealed sample bottle, and held at 37°C for 24 hours in this state, to obtain a test piece for a bending test.

### <Preparation of Test Piece for Bending Test by Thermal polymerization>

TEGDMA (10 parts by mass) was added to each monomer (specifically, each monomer shown in the column with a heading "30 (parts by mass)" in Table 5) (30 parts by mass) to be evaluated, and the mixture was stirred at 50°C until it became uniform, thereby obtaining a monomer composition.

LUPEROX 531M80 (0.32 parts by mass) was added to the obtained monomer composition, and the mixture was stirred at room temperature until it became uniform. 60 parts by mass of silica glass (SCHOTT AG, GM8235) as a filler was further added thereto, and the mixture was stirred and defoamed using a stirring defoaming device (MAZERUSTAR, manufactured by Kurabo Industries Ltd.), to obtain a curable composition for thermal polymerization.

The obtained curable composition for thermal polymerization was placed in a screw tube bottle (FC Screw Tube No. 7), and the monomers in the curable composition were thermally polymerized for 40 hours under the condition of a maximum reached temperature of 115°C using a bench-top type temperature chamber (SU-222, manufactured by ESPEC Corp.) to thermally cure the curable composition, and obtain a cured product A. The obtained cured product A was taken out from the screw tube bottle and further cured at 130°C for 2 hours using the bench-top type temperature chamber, to obtain a cured product B. A test piece of 2 mm × 2 mm × 25 mm was cut out from the obtained cured product B using a precision cutting machine (PRESISO CL-40, SANKEI Co., Ltd.). The obtained test piece was placed in the bench-top type temperature chamber and heat-treated at 130°C for 1 hour. Thereafter, the test piece was immersed in distilled water in a sealed sample bottle, and held at 37°C for 24 hours in this state, to obtain a test piece for a bending test.

### <Bending Test>

The test piece for a bending test obtained in either the aforementioned "Preparation of Test Piece for Bending Test by Photopolymerization" or the aforementioned "Preparation of Test Piece for Bending Test by Thermal polymerization" was subjected to a three-point bending test using a tester (AUTOGRAPH EZ-S manufactured by Shimadzu Corporation) under the conditions of a distance between supporting points of 20 mm and a crosshead speed of 1 mm/min, and the maximum stress (MPa) and the elastic modulus (MPa) were measured.

### <Preparation of Thiourethane (Meth)Acrylate Compound>

Monomers 1 to 22, which are specific examples of the thiourethane (meth)acrylate compound of the present disclosure, were prepared as follows.

### (Preparation of Monomer 1)

In a 100 mL four-necked flask that had been sufficiently dried and that was equipped with a stirring blade and a thermometer, DiBTSn (0.1 parts by mass), TMXDI (23.40 parts by mass) as the isocyanate compound (D), BHT (0.05 parts by mass), and T1 (1.75 parts by mass) as the thiol compound (B) were charged and dissolved to obtain a homogeneous solution. The obtained homogeneous solution was allowed to react at 80°C for 4 hours, to obtain a solution containing an intermediate.

The obtained solution was heated to 90°C, and 4HBA (24.86 parts by mass) as the (meth)acrylate compound (C) was added dropwise thereto over 1 hour. Since the internal temperature increased during the dropwise addition due to reaction heat, the dropwise addition quantity was controlled so that the temperature was 90°C or lower. After the total amount (24.86 parts by mass) of 4HBA was added dropwise, the reaction was allowed to proceed for 10 hours while the reaction temperature was maintained at 90°C. During the reaction, the progress of the reaction was tracked by HPLC analysis, and the completion of the reaction was confirmed. The product was discharged from the reactor, to obtain a composition (50 g) containing Monomer 1, which is a specific example of the thiourethane (meth)acrylate compound of the present disclosure.

Table 1 shows the respective types and amounts of the isocyanate compound (D), the thiol compound (B), and the (meth)acrylate compound (C), and further shows the molar ratio (hereinafter also referred to as "SH/NCO") of the amount of mercapto groups (SH groups) in the charge amount of the thiol compound (B) to the amount of isocyanate groups (NCO groups) in the charge amount of the isocyanate compound (D).

### (Monomers 2 to 22)

Compositions (50 g each) respectively containing Monomers 2 to 22 were obtained in the same manner as that in the preparation of Monomer 1, except that the types and amounts of the isocyanate compound (D), the thiol compound (B), and the (meth)acrylate compound (C) were respectively changed as shown in Tables 1 and 2.

**Table 1**

| Monomer No. | 1 | 2 | 3 | 4 | 5 | 6 | 7 | 8 | 9 | 10 | 11 |
|---|---|---|---|---|---|---|---|---|---|---|---|
| Isocyanate (D) (parts by mass) | TMXDI | TMXDI | TMXDI | TMXDI | TMXDI | TMXDI | TMXDI | TMXDI | TMXDI | TMXDI | NBDI |
| | 23.40 | 22.32 | 23.40 | 24.59 | 25.90 | 22.57 | 23.67 | 23.67 | 25.92 | 25.60 | 21.31 |
| Thiol (B) (parts by mass) | T1 | T1 | T1 | T1 | T1 | T2 | T2 | T2 | T3 | T4 | T1 |
| | 1.75 | 1.67 | 1.75 | 1.83 | 1.93 | 1.13 | 1.18 | 1.18 | 1.89 | 2.50 | 1.88 |
| (Meth)acrylate (C) (parts by mass) | 4HBA | 2HBMA | HPMA | HPA | HEA | 2HBMA | 4HBA | HPMA | HEA | HEA | 4HBA |
| | 24.86 | 26.01 | 24.86 | 23.58 | 22.17 | 26.30 | 25.14 | 25.14 | 22.18 | 21.90 | 26.81 |
| SH/NCO (molar ratio) | 0.10 | 0.10 | 0.10 | 0.10 | 0.10 | 0.10 | 0.10 | 0.10 | 0.10 | 0.10 | 0.10 |

**[Table 2]**

| Monomer No. | 12 | 13 | 14 | 15 | 16 | 17 | 18 | 19 | 20 | 21 | 22 |
|---|---|---|---|---|---|---|---|---|---|---|---|
| Isocyanate (D) (parts by mass) | NBDI | NBDI | NBDI | NBDI | NBDI | NBDI | XDI | XDI | XDI | TMHDI | TMHDI |
| | 22.29 | 22.48 | 23.79 | 22.48 | 23.48 | 23.50 | 19.97 | 21.17 | 21.08 | 23.72 | 23.66 |
| Thiol (B) (parts by mass) | T1 | T1 | T1 | T1 | T4 | T5 | T1 | T1 | T4 | T4 | T4 |
| | 0.98 | 1.99 | 2.10 | 1.99 | 2.71 | 2.60 | 0.97 | 1.02 | 2.67 | 2.69 | 8.04 |
| (Meth)acrylate (C) (parts by mass) | HPA | HPA | HEA | HEMA | HEA | HEA | HPMA | HPA | HPA | HEA | HEA |
| | 26.73 | 25.53 | 24.11 | 25.53 | 23.80 | 23.85 | 29.06 | 27.81 | 26.24 | 23.58 | 18.29 |
| SH/NCO (molar ratio) | 0.05 | 0.10 | 0.10 | 0.10 | 0.10 | 0.10 | 0.05 | 0.05 | 0.10 | 0.10 | 0.30 |

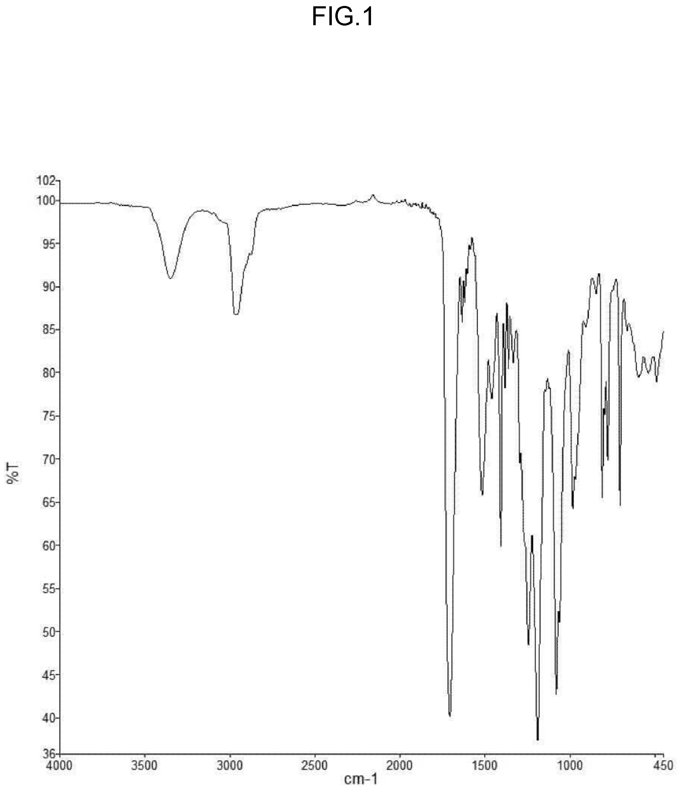

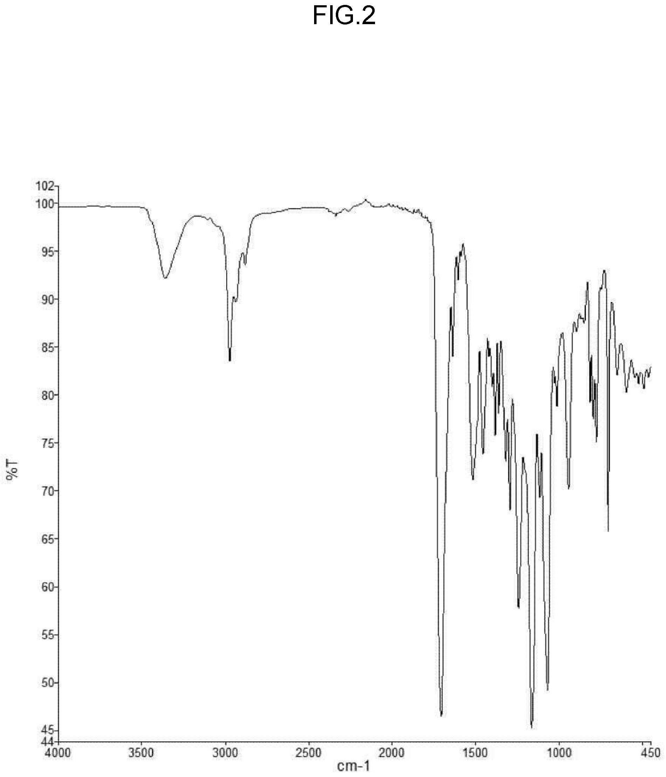

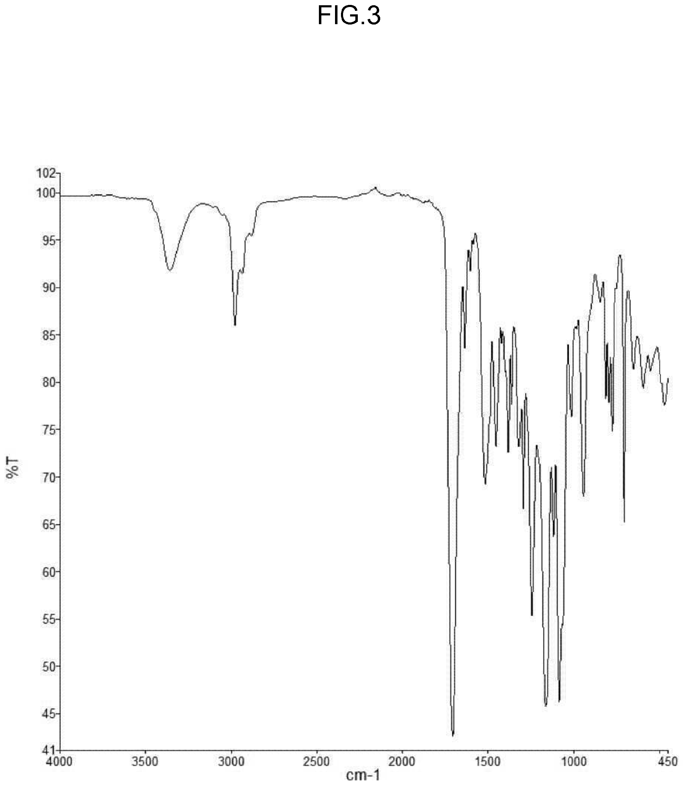

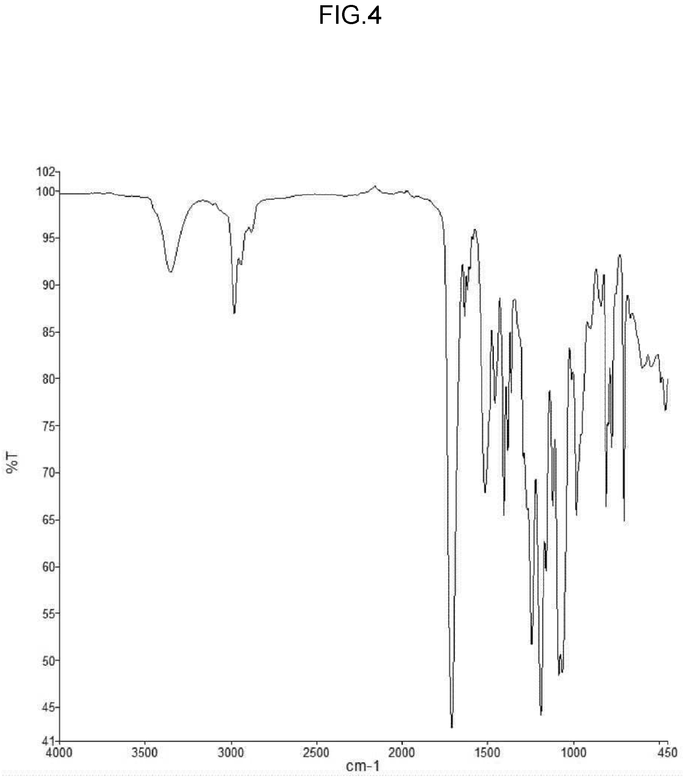

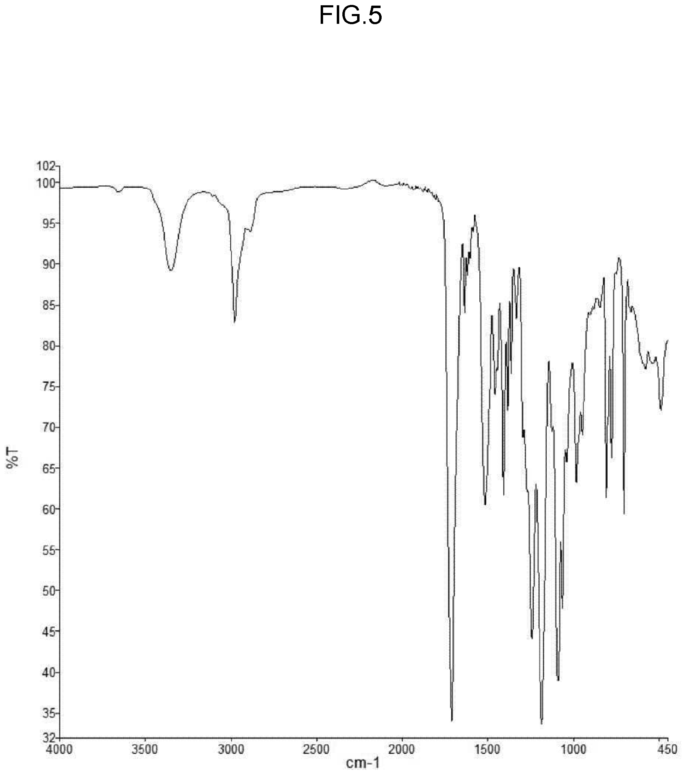

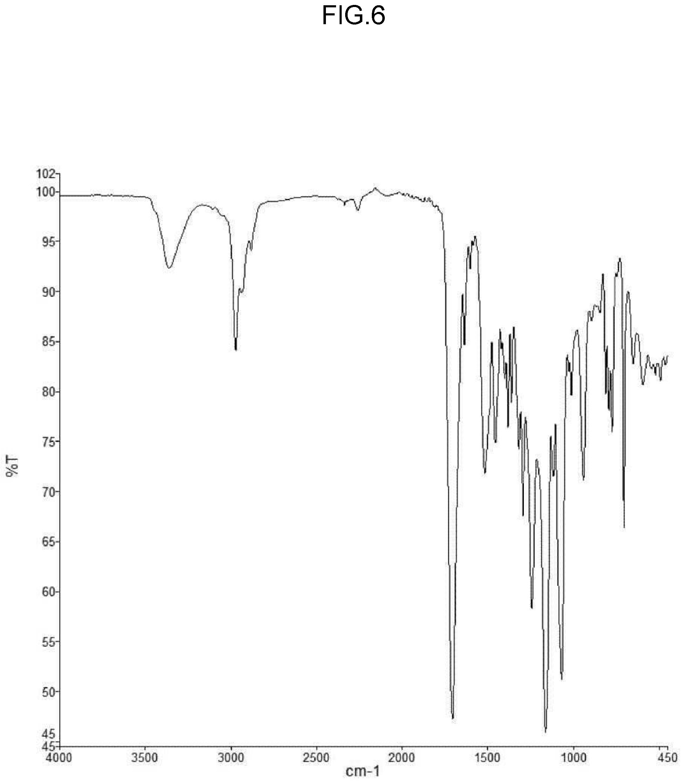

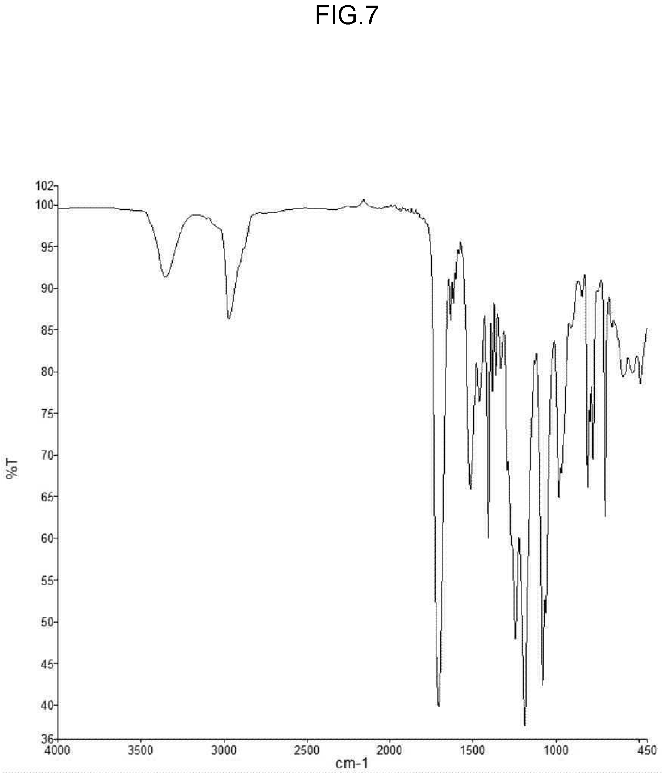

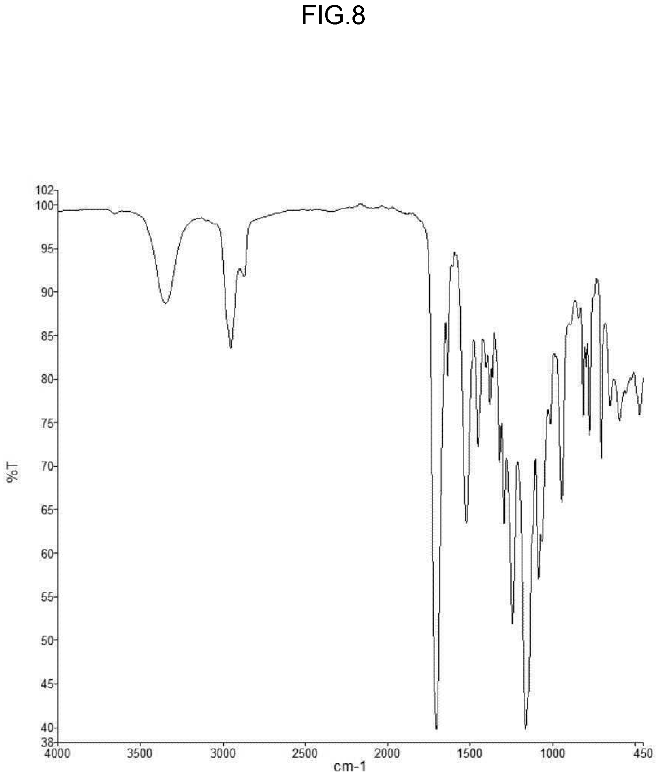

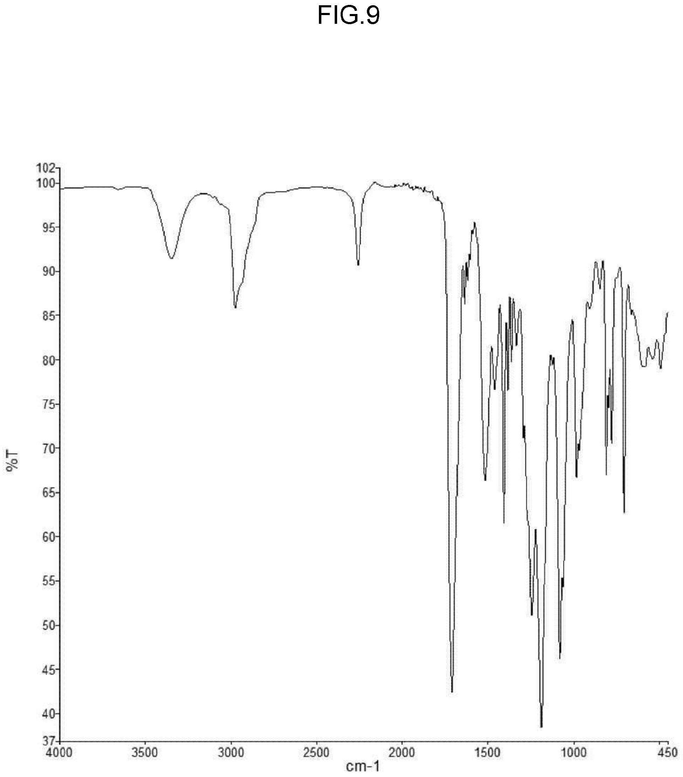

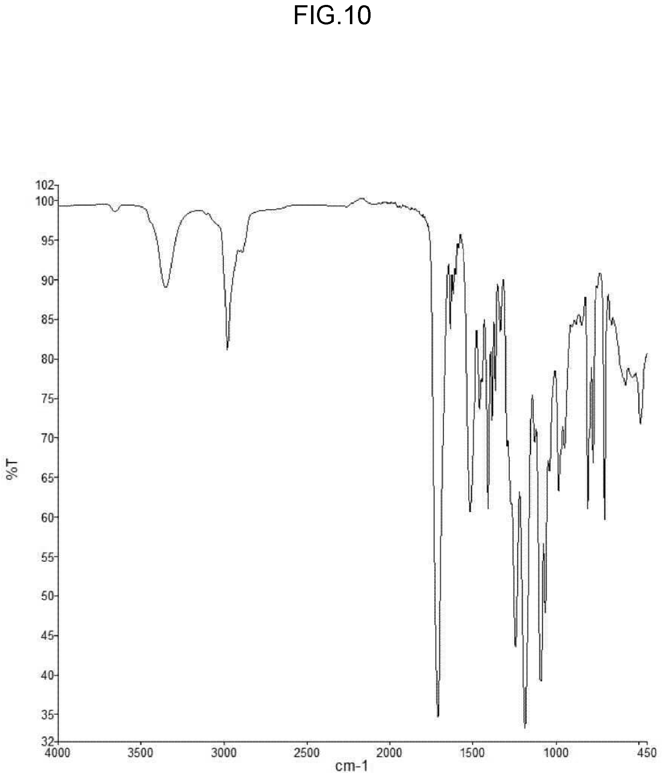

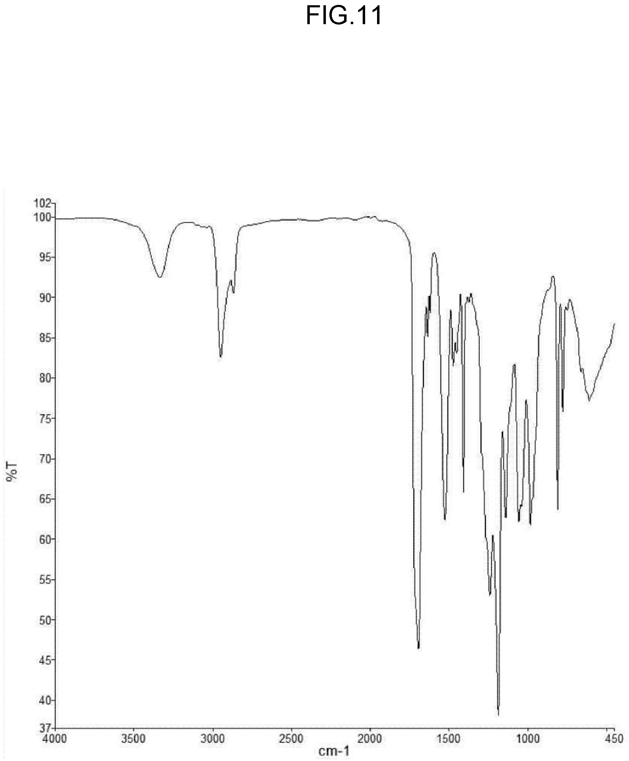

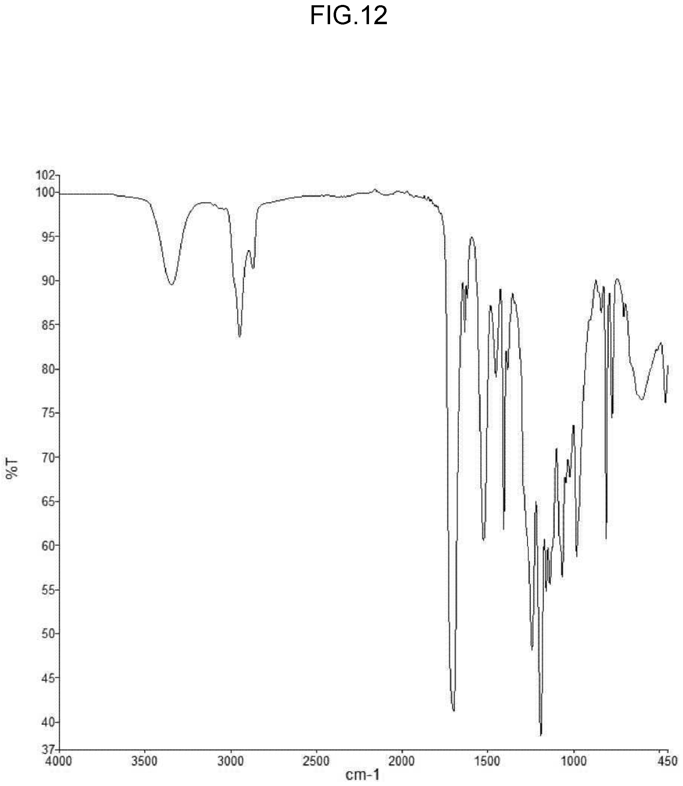

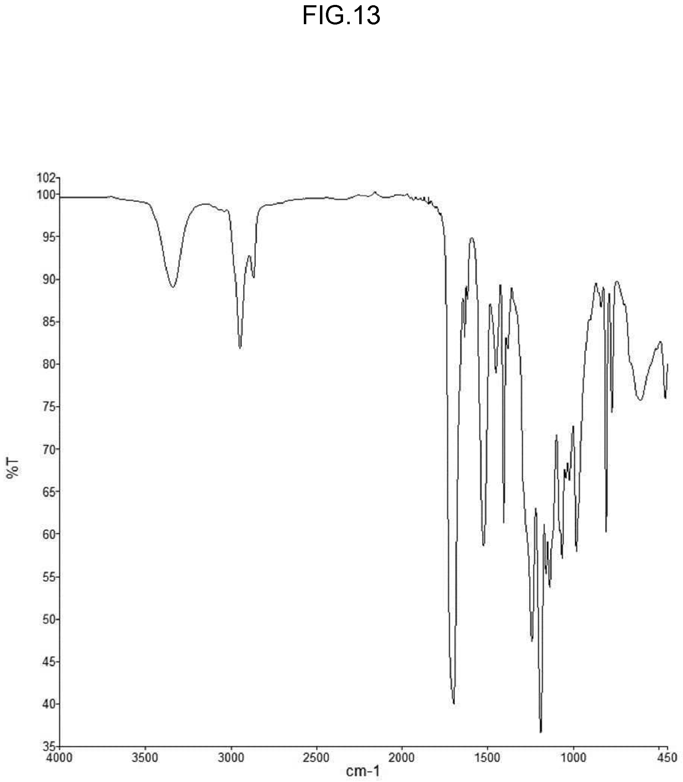

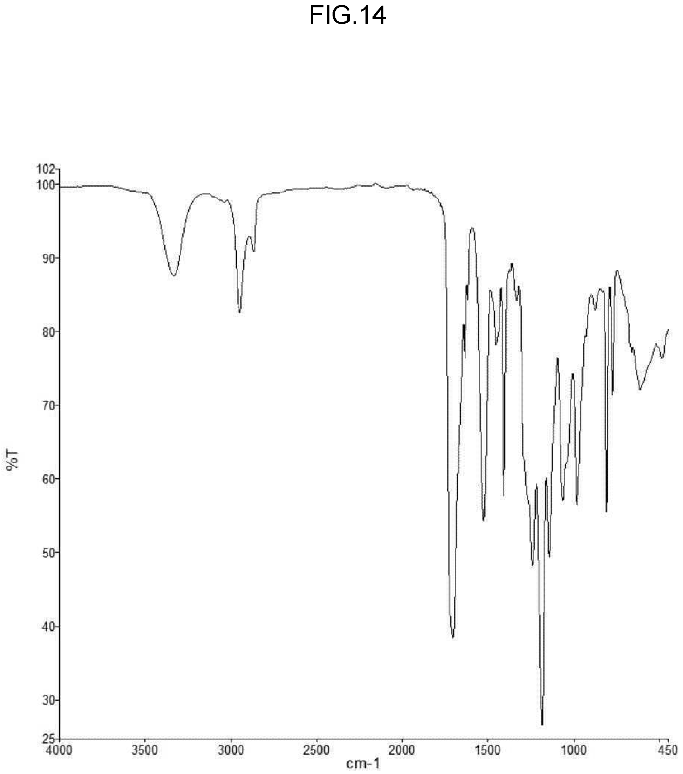

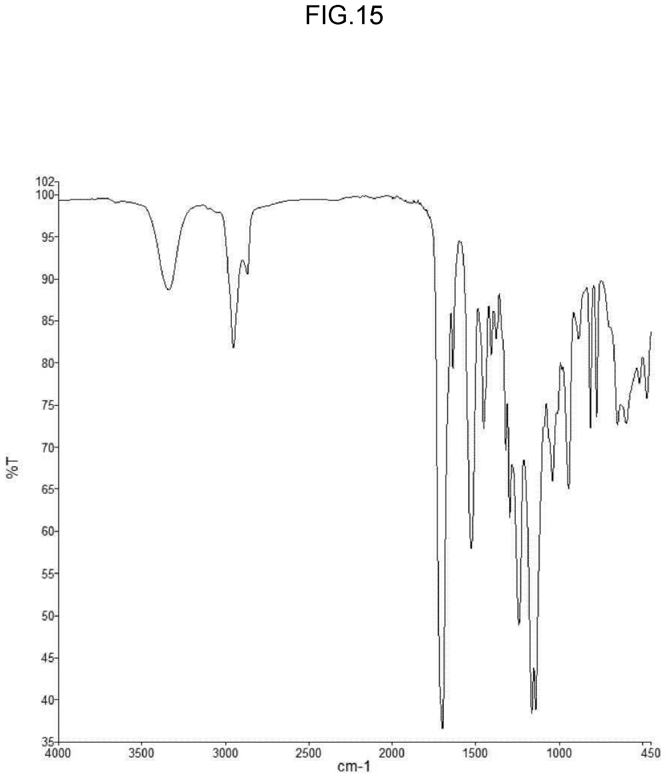

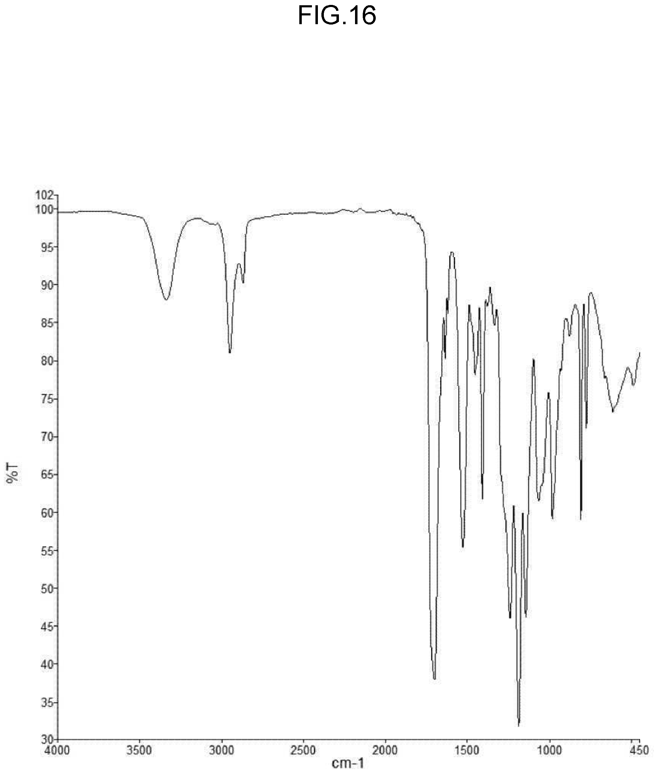

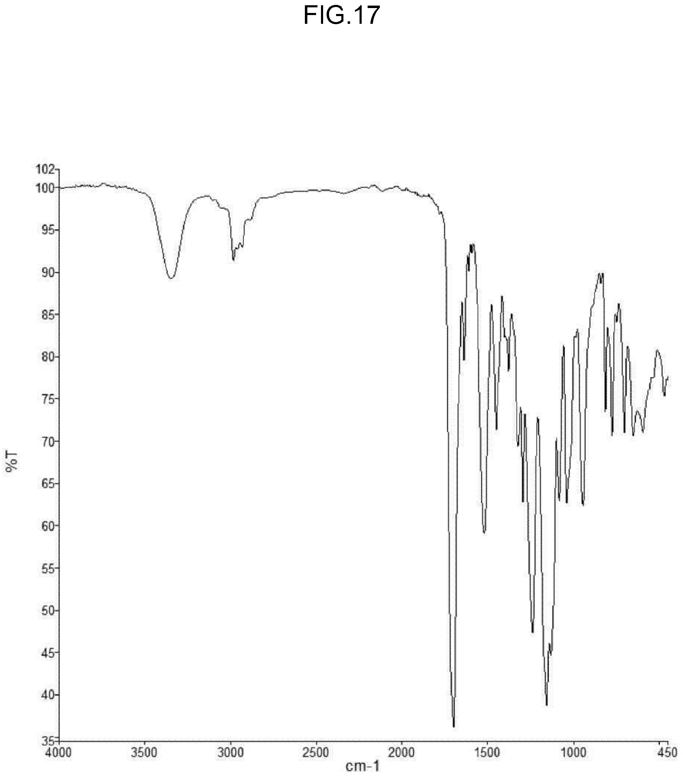

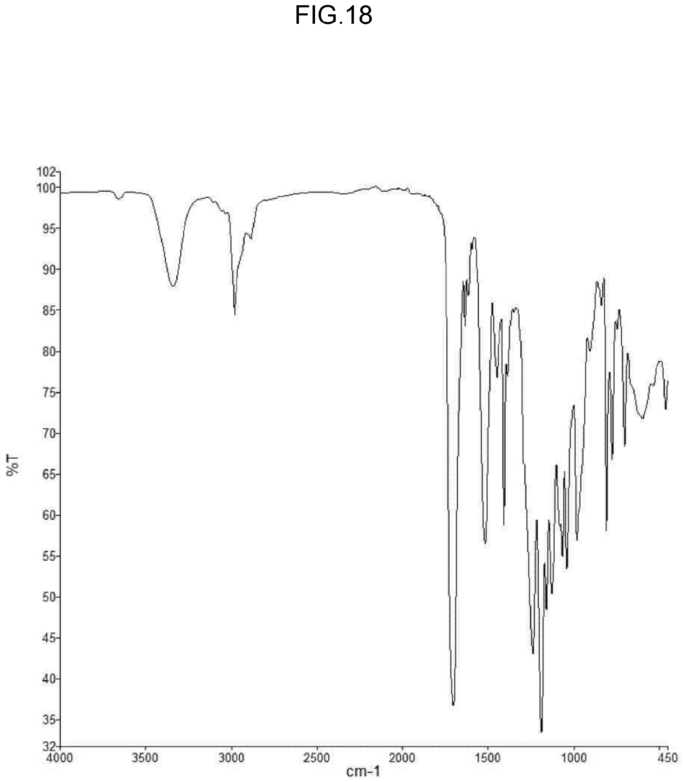

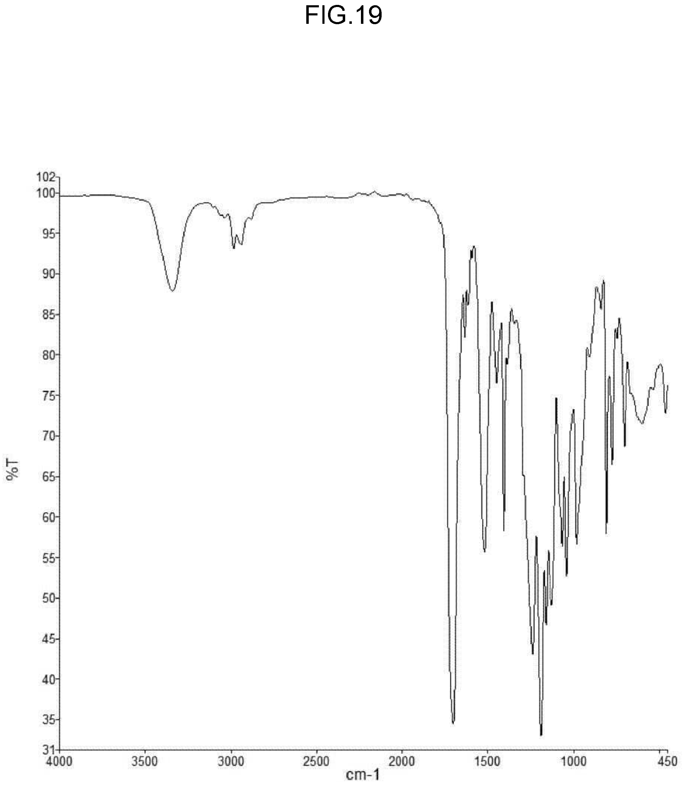

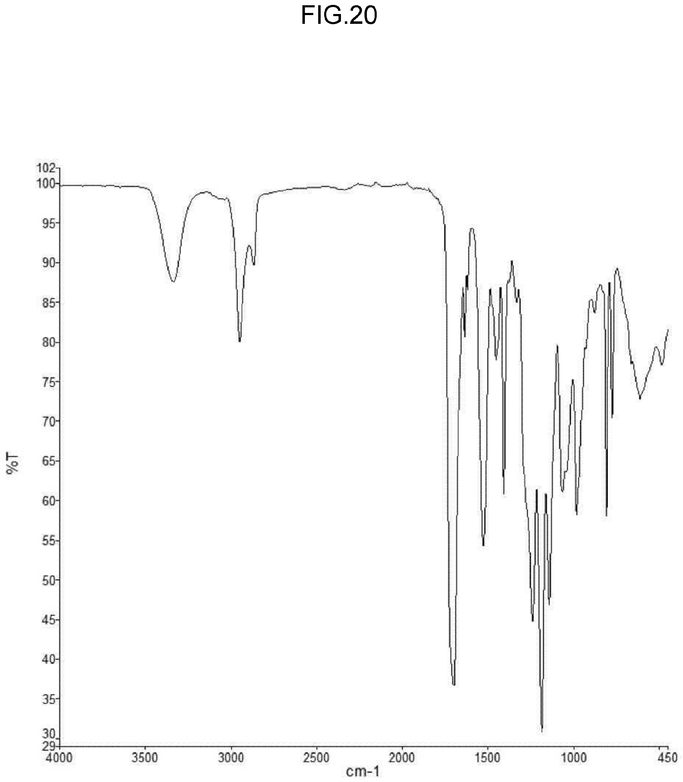

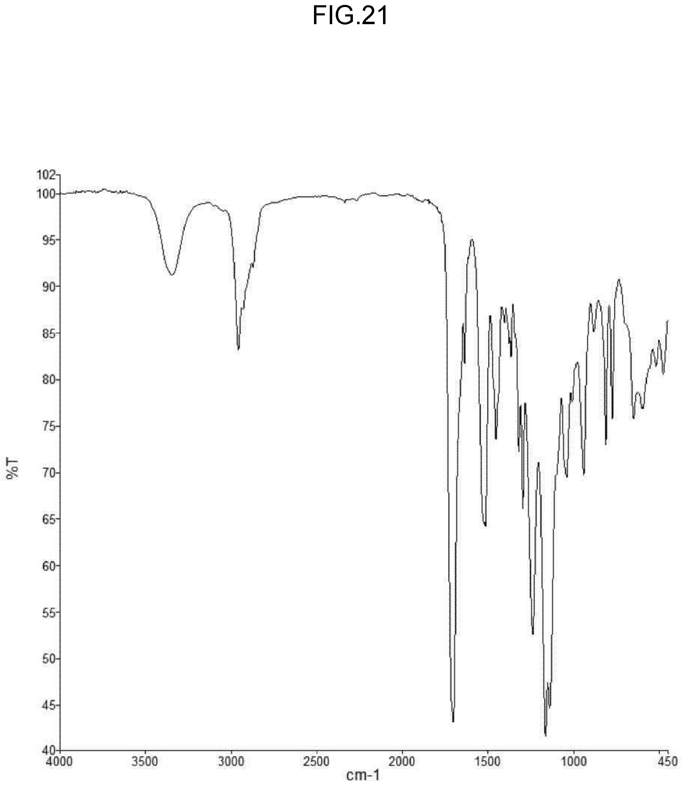

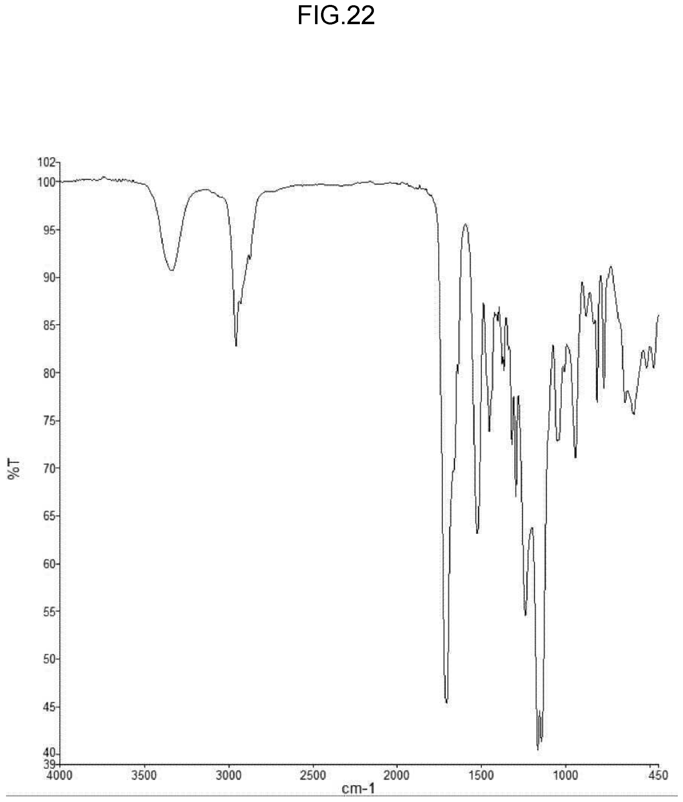

IR spectra (i.e., infrared absorption spectra) of Monomers 1 to 22 are shown in Figs. 1 to 22, respectively.

These IR spectra were measured under a temperature condition of 20°C.

As a result of these IR spectra, it was confirmed that all of Monomers 1 to 22 contained the compound represented by Formula (A1) described above.

In the compound represented by Formula (A1) contained in each of Monomers 1 to 22,
R^{1A} is a residue obtained by removing two mercapto groups from the thiol (B) shown in Tables 1 and 2,
R^{2A} is a residue obtained by removing two isocyanate groups from the isocyanate (D) shown in Tables 1 and 2, and
R^{3A} is a residue obtained by removing one hydroxy group and one (meth)acryloyloxy group from the (meth)acrylate (C) shown in Tables 1 and 2.

Among Monomers 1 to 22, monomers in which the (meth)acrylate (C) is an acrylate have R^{4A} representing a hydrogen atom, and monomers in which the (meth)acrylate (C) is a methacrylate have R^{4A} representing a methyl group.

Infrared absorption spectra of Monomers 1 to 22 are shown in Figs. 1 to 22, respectively.

These infrared absorption spectra were measured under a temperature condition of 20°C.

### [Comparative Example 1 and Examples 1 to 16]

Using the monomers shown in the column with a heading "6 (parts by mass)" in the following Tables 3 and 4, curable compositions for photopolymerization and test pieces for a bending test were prepared according to the foregoing "Preparation of Test Piece for Bending Test by Photopolymerization", and the foregoing "Bending Test" was performed using the obtained test pieces for a bending test.

The results are shown in Tables 3 and 4.

**Table 3**

| | | Comparative Example 1 | Example 1 | Example 2 | Example 3 | Example 4 | Example 5 | Example 6 | Example 7 | Example 8 |
|---|---|---|---|---|---|---|---|---|---|---|
| Monomer composition (for photopolymerization) | 6 (parts by mass) | UDMA | Monomer 1 | Monomer 2 | Monomer 3 | Monomer 4 | Monomer 5 | Monomer 6 | Monomer 7 | Monomer 12 |
| | 4 (parts by mass) | TEGDMA | TEGDMA | TEGDMA | TEGDMA | TEGDMA | TEGDMA | TEGDMA | TEGDMA | TEGDMA |
| Bending test evaluation result | Maximum stress (MPa) | 76 | 93 | 86 | 91 | 94 | 82 | 95 | 108 | 95 |
| | Elastic modulus (MPa) | 1510 | 2017 | 2376 | 2267 | 2064 | 2267 | 2110 | 2444 | 1903 |

**Table 4**

| | | Example 9 | Example 10 | Example 11 | Example 12 | Example 13 | Example 14 | Example 15 | Example 16 |
|---|---|---|---|---|---|---|---|---|---|
| Monomer composition (for photopolymerization) | 6 (parts by mass) | Monomer 13 | Monomer 14 | Monomer 15 | Monomer 16 | Monomer 17 | Monomer 18 | Monomer 19 | Monomer 20 |
| | 4 (parts by mass) | TEGDMA | TEGDMA | TEGDMA | TEGDMA | TEGDMA | TEGDMA | TEGDMA | TEGDMA |
| Bending test evaluation result | Maximum stress (MPa) | 95 | 97 | 88 | 99 | 106 | 90 | 82 | 86 |
| | Elastic modulus (MPa) | 1893 | 1967 | 1685 | 2088 | 2227 | 1930 | 1667 | 1734 |

As shown in Tables 3 and 4, cured products obtained by photopolymerization (i.e., photocuring) using the curable compositions for photopolymerization that contained respective monomers of Examples 1 to 16 exhibited bending strengths (specifically, maximum stress and elastic modulus) that were superior to those exhibited by a cured product obtained by photopolymerization (i.e., photocuring) using a curable compositions for photopolymerization that contained UDMA.

### [Comparative Example 101 and Examples 101 to 109]

Using the monomers shown in the column with a heading "30 (parts by mass)" in Table 5, curable compositions for thermal polymerization and test pieces for a bending test were prepared according to the foregoing "Preparation of Test Piece for Bending Test by Thermal polymerization", and the foregoing "Bending Test" was performed using the obtained test pieces for a bending test.

The results are shown in Table 5.

**Table 5**

| | | Comparative Example 101 | Example 101 | Example 102 | Example 103 | Example 104 | Example 105 | Example 106 | Example 107 | Example 108 | Example 109 |
|---|---|---|---|---|---|---|---|---|---|---|---|
| Composition (for thermal polymerization) | 30 (parts by mass) | UDMA | Monomer 5 | Monomer 8 | Monomer 9 | Monomer 10 | Monomer 11 | Monomer 14 | Monomer 19 | Monomer 21 | Monomer 22 |
| | 10 (parts by mass) | TEGDMA | TEGDMA | TEGDMA | TEGDMA | TEGDMA | TEGDMA | TEGDMA | TEGDMA | TEGDMA | TEGDMA |
| | 60 (parts by mass) | Silica glass | Silica glass | Silica glass | Silica glass | Silica glass | Silica glass | Silica glass | Silica glass | Silica glass | Silica glass |
| Bending test evaluation result | Maximum stress (MPa) | 127 | 213 | 176 | 177 | 215 | 151 | 187 | 207 | 184 | 185 |
| | Elastic modulus (MPa) | 5224 | 8590 | 8335 | 7519 | 9324 | 5831 | 9191 | 9214 | 6658 | 7965 |

As shown in Table 5, cured products obtained by thermal polymerization (i.e., thermal curing) using the curable compositions for thermal polymerization that contained respective monomers of Examples 101 to 109 exhibited bending strengths (specifically, maximum stress and elastic modulus) that were superior to those exhibited by a cured product obtained by thermal polymerization (i.e., thermal curing) using a curable composition for thermal polymerization that contained UDMA.

The disclosure of Japanese Patent Application No. 2024-015856 filed on February 5, 2024 is incorporated herein by reference in its entirety.

All documents, patent applications, and technical standards mentioned in the present specification are incorporated herein by reference to the same extent as if each individual document, patent application, or technical standard were specifically and individually indicated to be incorporated by reference.

## Claims

1. A thiourethane (meth)acrylate compound, comprising:
two bonds each represented by the following Formula (X);
two urethane bonds; and
two (meth)acryloyl groups: 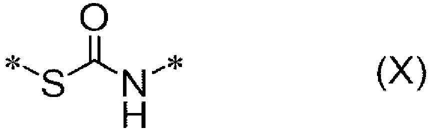
wherein, in Formula (X), each of two instances of * is a bonding position.

2. The thiourethane (meth)acrylate compound according to claim 1, which is a reaction product of a thiol compound (B) comprising two mercapto groups, a (meth)acrylate compound (C) comprising one hydroxy group and one (meth)acryloyloxy group, and an isocyanate compound (D) comprising two isocyanate groups.

3. The thiourethane (meth)acrylate compound according to claim 1, which is a compound represented by the following Formula (A1): 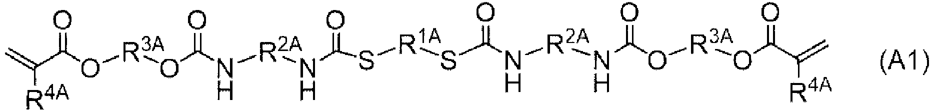 wherein, in Formula (AI):
R^{1A} is a residue obtained by removing two mercapto groups from a thiol compound (B) comprising two mercapto groups;
R^{2A} is a residue obtained by removing two isocyanate groups from an isocyanate compound (D) comprising two isocyanate groups;
R^{3A} is a residue obtained by removing one hydroxy group and one (meth)acryloyloxy group from a (meth)acrylate compound (C) comprising one hydroxy group and one (meth)acryloyloxy group;
R^{4A} is a hydrogen atom or a methyl group;
two instances of *R^{2A}* may be the same as or different from each other;
two instances of *R^{3A}* may be the same as or different from each other; and
two instances of *R^{4A}* may be the same as or different from each other.

4. The thiourethane (meth)acrylate compound according to claim 3, wherein the isocyanate compound (D) comprises a compound represented by any one of the following Formulae (D1) to (D8):

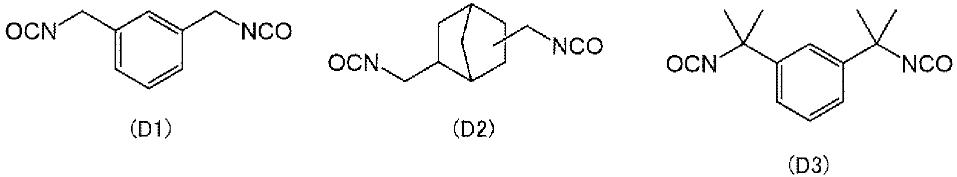

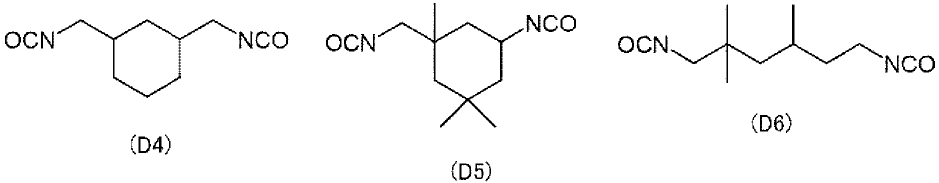

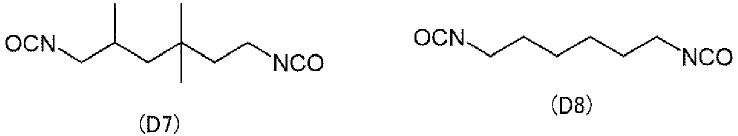

5. The thiourethane (meth)acrylate compound according to claim 3, wherein the thiol compound (B) comprises a compound represented by the following Formula (B1): 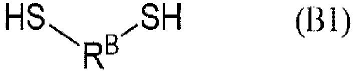 wherein, in Formula (B1), R^{B} is a divalent organic group having from 1 to 20 carbon atoms.

6. The thiourethane (meth)acrylate compound according to claim 3, wherein the (meth)acrylate compound (C) comprises a compound represented by the following Formula (C1): 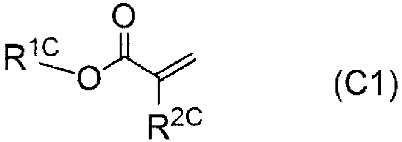 wherein, in Formula (C1):
R^{1C} is an organic group having from 2 to 25 carbon atoms and comprising one hydroxy group; and
R^{2C} is a hydrogen atom or a methyl group.

7. A monomer composition, comprising the thiourethane (meth)acrylate compound according to any one of claims 1 to 6.

8. The monomer composition according to claim 7, wherein the monomer composition is for a dental material.

9. A curable composition, comprising the thiourethane (meth)acrylate compound according to any one of claims 1 to 6, and a polymerization initiator.

10. A dental material, comprising the curable composition according to claim 9 or a cured product of the curable composition.
